# EUROPEAN PATENT SPECIFICATION

(11) **EP 3 538 081 B1**
(45) Date of publication and mention of the grant of the patent: **02.08.2023**
(21) Application number: 17811860.0
(22) Date of filing: 14.11.2017
(51) Int. Cl.: A61K 31/132, A61K 31/05, A61K 31/07, A61K 31/353, A61K 31/355, A61K 31/375, A61K 36/899, A61P 3/04, C12N 5/00

(54) **USE OF A SPERMIDINE COMPRISING EXTRACT FOR THE ENHANCEMENT OF MITOCHONDRIAL RESPIRATION**
VERWENDUNG VON EINEM SPERMIDIN ENTHALTENDEN EXTRAKT ZUR VERBESSERUNG DER MITOCHONDRIALEN ATMUNG
UTILISATION D'UN EXTRAIT COMPRENANT DE LA SPERMIDINE POUR L'AMÉLIORATION DE LA RESPIRATION MITOCHONDRIALE

(30) Priority: 14.11.2016 EP 16198663
(43) Date of publication of application: 18.09.2019
(73) Proprietor: TLL The Longevity Labs GmbH, 8020 Graz (AT)
(72) Inventor: MADEO, Frank, 8044 Graz (AT); EISENBERG, Tobias, 8010 Graz (AT); STEKOVIC, Slaven, 8010 Graz (AT)
(74) Representative: Pföstl, Andreas
(86) International application number: PCT/EP2017/079180
(87) International publication number: WO 2018/087388

(56) References cited:
- WO-A1-2007/148739
- WO-A1-2016/149277
- WO-A2-03/051348
- WO-A2-2010/081204
- WO-A2-2010/081862
- US-A1- 2014 378 708
- US-A1- 2014 378 708
- SABRINA BÜTTNER ET AL: "Spermidine protects against [alpha]-synuclein neurotoxicity", CELL CYCLE, vol. 13, no. 24, 31 October 2014 (2014-10-31), pages 3903-3908, XP055367093, US ISSN: 1538-4101, DOI: 10.4161/15384101.2014.973309
- MINOIS NADEGE ET AL: "Spermidine Feeding Decreases Age-Related Locomotor Activity Loss and Induces Changes in Lipid Composition", PLOS ONE, vol. 9, no. 7, E102435, July 2014 (2014-07), pages 1-11, XP002769682,
- SOULET D ET AL: "Role of endocytosis in the internalization of spermidine-C2-BODIPY, a highly fluorescent probe of polyamine transport", BIOCHEMICAL JOURNAL, vol. 367, no. 2, 15 October 2002 (2002-10-15), pages 347-357, XP002769683, DOI: 10.1042/BJ20020764
- HIROSE TADAO ET AL: "Spermidine and Ca2+, but not Na+, can permeate NMDA receptors consisting of GluN1 and GluN2A or GluN2B in the presence of Mg2+", BIOCHEMICAL AND BIOPHYSICAL RESEARCH COMMUNICATIONS, vol. 463, no. 4, August 2015 (2015-08), pages 1190-1195, XP002769684,
- MINOIS NADEGE: "Molecular Basis of the 'Anti-Aging' Effect of Spermidine and Other Natural Polyamines A Mini-Review", GERONTOLOGY, vol. 60, no. 4, 2014, pages 319-326, XP002769685,
- MINOIS NADEGE ET AL: "Polyamines in aging and disease", AGING-US, vol. 3, no. 8, August 2011 (2011-08), XP002769686, ISSN: 1945-4589
- BROWN L ET AL: "Fermented wheat germ extract (avemar) in the treatment of cardiac remodeling and metabolic symptoms in rats", EVIDENCE-BASED COMPLEMENTARY AND ALTERNATIVE MEDICINE, OXFORD UNIVERSITY PRESS, US, vol. 2011, 1 January 2011 (2011-01-01), page ArticleID508957, 10pp, XP009160264, ISSN: 1741-427X, DOI: 10.1093/ECAM/NEP090
- Jung-Hee Jang ET AL: "Neuroprotective effects of Triticum aestivum L. against Î2-Amyloid-induced cell death and memory impairments", PHYTOTHERAPY RESEARCH., vol. 24, no. 1, 1 January 2010 (2010-01-01), pages 76-84, XP055695044, GB ISSN: 0951-418X, DOI: 10.1002/ptr.2871
- ZHU K ET AL: "In vitro study on anti-breast cancer activity of wheat germ water soluble extract", JOURNAL OF THE CHINESE CEREALS AND OILS ASSOCIATION JULY 2013 EDITOR. DEPT. OF J. OF THE CHINESE CEREALS AND OILS ASSOC. CHN, vol. 28, no. 7, July 2013 (2013-07), pages 19-22+28, XP009520403,
- Kazuhiro Nishimura ET AL: "Decrease in Polyamines with Aging and Their Ingestion from Food and Drink", Journal of Biochemistry, vol. 139, no. 1, 1 January 2006 (2006-01-01), pages 81-90, XP055692329, GB ISSN: 0021-924X, DOI: 10.1093/jb/mvj003

## Description

### Technical Field

The present invention relates to the field of polyamines having mitochondrial respiratory activity enhancing properties.

### Background Art

Spermidine (N-(3-Aminopropyl)-1,4-diaminobutane) is a polycationic aliphatic amine which plays several roles in cell survival. Spermidine is produced from putrescine by spermidine synthase using an aminopropyl group from decarboxylated S-adenosyl-L-methionine (SAM).

Spermidine is well-known to be a longevity agent in mammals due to various mechanisms of action such as induction of autophagy (Eisenberg et al. Nat Cell Biol. 11(2009):1305-14), reduction of inflammation, lipid metabolism and regulation of cell growth, proliferation and death. The effect has caused this substance to become one of the standard autophagy activators and caloric restriction mimetics in preclinical life science and biomedi-cine. For example, WO 2010/081204 discloses polyamines such as spermidine that activate the autophagic pathway which can be used to increase the average lifespan of animals. As a further example, WO 2010/081862 discloses the use of polyamines, in particular spermidine for the treatment of patients suffering a life-threatening condition such as lactic acidosis, muscle weakening or multiple organ failure. Another publication (Büttner et al. (Cell Cycle 13(2014):3903-3908)) describes the positive effects of spermidine on the course of Parkinson's disease by the induction of autophagy. A further example is disclosed in WO 03/051348. This patent application describes polyamines and methods for their preparation that can be used in the treatment of degenerative diseases associated with mitochondrial dysfunction, as e.g., Parkinson's disease or Alzheimer's disease. A further publication describes a mechanism of action for spermidine-induced autophagy (Minois et al. (PLOS ONE 9(2014):e102435)). In this work, the authors show that spermidine improves the motility of aged flies demonstrating the effects of spermidine on aging. Yet another publication describes the uptake characteristics spermidine in CHO cells (Soulet D et al. (Biochem J 367(2002):347-357)). Yet, another publication describes the transport of spermidine through membrane channels of HEK293 cells (Hirose T et al. (Biophys Res Comm 463(2015):1190-1195)) .

### Summary of invention

While most research has concentrated on the application of spermidine as a longevity substance, it turned now out that spermidine plays an important role in the mitochondrial regulation as a potential performance enhancer and a tool to enhance antioxidative effects of various substances.

It turned surprisingly out that spermidine is capable of increasing respiratory activity, in particular the Complex I activity, in mammalian mitochondria which leads to an increased production of energy in the cell. This allows using spermidine for various purposes including complex I pathologies and increasing tolerance to physical activity and exercise. Since Complex I is one of the main sites at which premature electron leakage to oxygen occurs, the improvement of its efficiency through spermidine can also be applied for enhancement of antioxidant treatments to reduce reactive oxygen species (ROS) stress by preventing its generation and accumulation in the first place. Surprisingly, spermidine reduces the accumulation of ROS in mitochondria although the respiratory activity is increased.

Hence, the present invention relates to the non-therapeutic use according to claim 1.

Disclosed herein is spermidine or a spermidine comprising extract for use in the treatment and/or amelioration of a mitochondrial energy disorder or disease.

The treatment and/or amelioration is mediated via activation of mitochondrial complex I.

A mitochondrial energy disorder can be mitochondrial myopathy, encephalopathy, obesity or an obesity-related disorder.

Myopathy or encephalopathy is caused by a genetic mutation in mitochondrial complex I protein.

According to another preferred embodiment of the present invention the spermidine comprising extract is formulated for oral or topical administration.

The spermidine comprising extract is preferably formulated in a capsule, as a tablet, as powder, in a gel or in an ointment.

Spermidine is preferably administered in an amount of 1 pg/kg body weight per day to 500 mg/kg body weight per day, preferably 5 pg/kg body weight per day to 200 mg/kg body weight per day, more preferably 10 pg/kg body weight per day to 100 mg/kg body weight per day.

Also disclosed is a method of enhancing mitochondrial respiratory activity in a subject (i.e. human subject, human) or a mammal comprising administering spermidine or a spermidine comprising extract to said subject or mammal.

The enhancement of the mitochondrial respiratory activity is preferably mediated via activation of mitochondrial complex I.

According to a preferred embodiment of the present invention the enhancement of mitochondrial respiratory activity increases the mitochondrial energy production (ATP) and the efficiency of mitochondrial energy production.

The enhancement of mitochondrial respiratory activity results preferably in a reduced, substantially reduced or substantially identical formation of reactive oxygen species (ROS) .

According to another preferred embodiment of the present invention the enhancement of mitochondrial respiratory activity enhances function of skeletal muscle and/or endurance and/or decreases white adipose tissue, increases formation of brown adipose tissue, transforms white adipose tissue into brown adipose tissue or enhances lipolysis.

The mammal to which spermidine or a spermidine comprising extract is preferably administered is preferably a horse, a camel, a dog, a cat or a rodent.

Disclosed is also a method of enhancing energy and muscle and/or sports performance in a subject or a mammal comprising administering spermidine to said subject or mammal.

According to a preferred embodiment of the present invention at least one antioxidant is administered before, after or together with the spermidine comprising extract.

According to a further preferred embodiment of the present invention the antioxidant is selected from the group consisting of ascorbic acid, α-Tocopherol, tocotrienol, retinol, polyphenols and derivatives thereof.

According to another preferred embodiment of the pre-sent invention the polyphenol is selected from the group consisting of quercetin, myricetin, catechin, theaflavin, peonidin, cyanidin, glycitein, isoflavones, resveratrol, pterostilbene, curcumin, tannins, vanillin and chlorogenic acid.

Disclosed is the use of spermidine for enhancing energy production and muscle and/or sports performance in a subject or a mammal

Another aspect of the present invention relates to a method of enhancing mitochondrial respiratory activity in a non-plant cell in vitro comprising incubating said non-plant cell in a culture medium comprising a spermidine comprising extract, wherein the spermidine comprising extract is extracted from wheat or wheat germs and wherein the culture medium comprises spermidine in a concentration ranging from 0.1 mmo1/1 to 100 mmol/l, preferably 0.1 mmo1/1 to 50 mmo1/1, more preferably 0.1 mmol/1 to 10 mmo1/1.

According to a preferred embodiment of the present invention the non-plant cell is a mammalian cell, preferably a human cell, a CHO cell, a HEK-293 cell, a BHK-21 cell, a NSO cell, a Sp2/0-Ag14 cell, a fungal cell or bacterial cell.

According to another preferred embodiment of the present invention the mitochondrial respiratory activity is utilized for yield optimization in production of native or recombinant proteins.

The protein to be expressed by incubating cells in a medium comprising a spermidine comprising extract is an enzyme, an antibody or a functional fragment thereof.

The spermidine comprising extract is extracted from wheat or wheat germs.

### Brief description of the figures

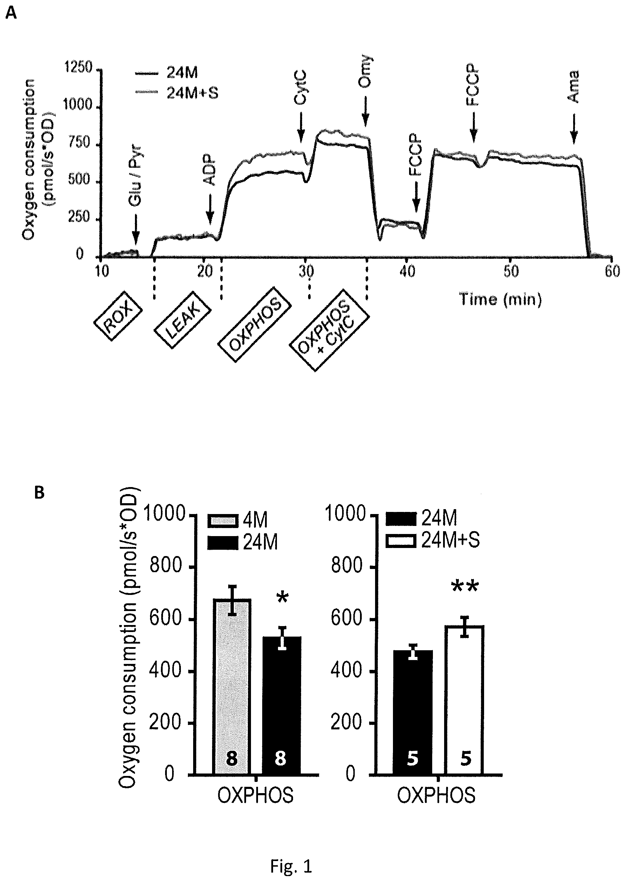

Fig. 1 shows the influence of spermidine on the activity of mitochondrial Complex I.

### Description of embodiments

Mitochondria are cellular organelles that are responsible for the most of the energy production in the cell. From the total of max. 32 ATP molecules produced by degrading one molecule glucose through glycolysis and oxidative phosphorylation, 28 ATP molecules are produced in mitochondria in the process of mitochondrial respiration. Mitochondrial respiration is a process of degradation of pyruvate molecules under consumption of oxygen, thereby creating proton gradient across the mitochondrial membrane. This allows for powering ATP synthase in order to produce high-energy ATP molecules from inorganic phosphate and ADP. ATP is further used as an energy source for chemical reactions in the cell.

The mitochondrial respiration happens in three steps:
1) Starting from Complex I or Complex II depending on the available substrate, ubiquinone (Q) is reduced to ubiquinol (QH2);
2) in Complex III, electrons are transported from QH₂ to the cytochrome c (Cyt c)
3) Complex IV transfers electrons from cytochrome c to a molecular oxygen, which in the end results in generation of water molecules.

The electron flow through Complex II is dependent on the substrate (succinate) level in the mitochondrion. The Complex I activity is dependent on the concentration of redox substrate NADH - a result of pyruvate degradation in the course of Krebs cycle. Therefore, the activity of these two enzyme complexes (routes of energy production in the mitochondrion) is independent of each other and determinant for the total amount of energy produced from initial glucose molecule.

Within Complex I (EC 1.6.5.3), two electrons are transferred from NADH to a lipid-soluble ubiquinone. The reduced product, ubiquinol, freely diffuses within the membrane. In parallel, Complex I translocates four protons (H+) across the membrane resulting in a proton gradient. This route produces more energy than the Complex II generated electron Pool.

Spermidine is surprisingly able to increase respiratory activity, in particular the Complex I activity, in mammalian mitochondria which leads to an increased production of energy in the cell. This allows using spermidine for various purposes including complex I pathologies and increasing tolerance to physical activity and exercise. Surprisingly, spermidine reduces the accumulation of ROS in mitochondria although the respiratory activity is increased.

Disclosed herein is spermidine or a spermidine comprising extract for use in the treatment and/or amelioration of a mitochondrial energy disorder or disease.

Furthermore, the use of spermidine or a spermidine comprising extract for manufacturing a medicament for treating and/or ameliorating a mitochondrial energy disorder or disease is disclosed.

According to the present invention the spermidine comprising extract can be provided in a dietary supplement. Spermidine may be formulated to this preparation in a pure or substantially pure (at least 95%, preferably at least 96%, more preferably at least 97%, more preferably at least 98%, more preferably at least 99%) form. Pure or substantially pure spermidine can be produced in vitro (Baxter et al., Biochem Biophys Res Commun. 1973,54(1):147-54) or in vivo (Tabor et al., J. Biol. Chem. 1958,233:907-914) by converting putrescine in the presence of spermidine synthase and S-adenosylmethioninamine or can be isolated from natural sources (e.g. legumes, grain, fungi). The isolation of spermidine from natural sources may be achieved by using methods known in the art including, for instance, treating grain, in particular milled grain, with trichloroacetic acid followed by an alkali treatment, alcohol extraction and chromatography (Minocha et al., J Plant Grow Regul 1994,13:187-193).

"Spermidine comprising extract", as used herein, refers to an extract from a spermidine comprising natural, preferably plant, source including legumes (e.g. soya) and grain (e.g. wheat) and germs thereof. "Spermidine comprising extracts" can be obtained by using various methods known in the art. The extract used in the present invention comprising spermidine as well as spermidine isolated from such an extract is preferably obtained from wheat or wheat germs. An extract as used in the present invention may comprise next to spermidine other substances which are naturally occurring in the source from which the extract is obtained or which are added during the extraction process. These substances may include also spermidine derivatives in a minor degree which may be formed during the extraction process or being present in the natural source.

In a first step the spermidine comprising source to be extracted may be macerated at least in part. Thereafter a solvent or a mixture of solvents, preferably a protic polar solvent, in particular water, an alcohol, a glycol, a polyol or a water/alcohol, water/glycol or water/polyol mixture of 100/0 to 0/100 (v/v).

These extracts are then preferably centrifuged and/or filtered and/or distilled in order to recover the active soluble fraction (crude extract). Additional steps of decolourisation and/or deodorization can be carried out on the extract at any stage of the extraction and according to techniques known by those skilled in the art.

The spermidine comprising extract of the present invention is preferably obtained by aqueous extraction, preferably in water alone. The extraction can be carried out for a period ranging from 1 hour to 20 hours, preferably for a period ranging from 2 hours to 16 hours, more preferably for a period ranging from 3 hours to 10 hours. The extraction can be carried out at a temperature ranging from 2 to 40°C, preferably ranging from 5 to 30°C, more preferably ranging from 10 to 25°C, in particular at room temperature (i.e. at 20 to 22°C).

The spermidine comprising source, which is preferably a plant source, can be macerated, grinded using, for instance, a bead mill, mortar grinded, ultrasonic grinded or grinded using a mixer.

A particularly preferred method for manufacturing a spermidine comprising extract from a natural source involves the use of water and ethanol as extraction media. An alternative method can be found in WO 2013/051483.

The spermidine comprising extract may comprise 0.001 wt% to 50 wt% (w/w; dry weight) spermidine, preferably 0.005 wt% to 40 wt% spermidine, more preferably 0.01 wt% to 30 wt% spermidine, more preferably 0.02 wt% to 20 wt% spermidine, more preferably 0.02 wt% to 10 wt% spermidine.

The spermidine comprising extract may comprise 10 pg/g to 500 mg/g (dry weight), preferably 50 pg/g to 400 mg/g, more preferably 100 pg/g to 300 mg/g, more preferably 200 pg/g to 200 mg/g, more preferably 200 pg/g to 100 mg/g, spermidine.

"Treatment" and "treating" of a disease or disorder, as used herein, refers to ameliorating and/or curing a disease as referred to herein, preventing Progression of the disease or at least an amelioration of at least one symptom associated with the said disease. A treatment as referred to herein will be effective in at least a statistically significant portion of the subjects that are treated.

As used herein, the term "amelioration" or "ameliorating" with reference to a disease, pathological condition or symptom, refers to any observable beneficial effect of the treatment. The beneficial effect can be evidenced, for example, by a delayed onset of clinical symptoms of the dis-ease in a susceptible subject, a reduction in severity of some or all clinical symptoms of the disease, a slower progression of the disease, a reduction in the number of relapses of the disease, an improvement in the overall health or well-being of the subject, or by other parameters well known in the art that are specific to the particular disease.

A "mitochondrial energy disorder or disease" or "mitochondrial energy metabolism disorder or disease", as used herein, refers to any disease or condition that is caused by or contributed to by mitochondrial abnormal function. Mitochondrial dysfunction is involved in a wide range of disorders. A mitochondrial disorder may arise at least in part from a mutation in a gene encoding a mitochondrial protein. In other instances a mitochondrial disorder may arise at least in part from a mutation in nuclear and/or mitochondrial DNA. The affected genes may encode for one or more proteins that regulate expression, localization, post-translational modification, activity or assembly of a mitochondrial protein or complex. Of course, mitochondrial disorders may also be characterized by mitochondrial dysfunctions that are not known to be attributable to mutations of a particular gene or genes. A "mitochondrial energy disorder or disease" may be characterized by an increased production of free radicals and reactive oxygen species that damage cells, in some cases resulting in cell or tissue loss.

A "mitochondrial energy disorder or disease" can also be the result of or an effect caused by another disease or disorder like obesity, renal failure, hypercholesterolaemia or hypertension or by smoking resulting in a depletion of ATP production or in an increase of ROS measured by the elevated amount of circulating products of lipid peroxidation, protein oxidation or metabolic byproducts of carbohydrate and DNA oxidation. These biomarkers include, but are not limited to IsoPs, MDA, Nitrotyrosine, S-glutahtionylation, MPO, OxLDL, ROS-induced changes in genetic transcription (measured by RNA microarrays) or serum antioxidative capacity (Ho et al., Redox Biol. 2013 Oct 8;1:483-91).

Spermidine can be used to treat and/or ameliorate mitochondrial energy disorders or diseases in human subjects and mammals. Mammals include horses, camels, dogs, cats or rodents like rabbits, mice and rats.

The treatment and/or amelioration of the mitochon-drial energy disorder or disease can be mediated via activation of mitochondrial complex I ("Online Mendelian Inheritance in Man" (OMIM) database accession number #252010).

Isolated deficiency of mitochondrial respiratory chain complex I can be caused by mutations in multiple different genes, both nuclear-encoded and mitochondrial-encoded. A complex I deficiency results from muta-tion in any of the subunits of complex I. A complex I deficiency results from mutation in nuclear-encoded subunit genes, including NDUFV1 (OMIM #161015), NDUFV2 (OMIM #600532), NDUFS1 (OMIM #157655), NDUFS2 (OMIM #602985), NDUFS3 (OMIM #603846), NDUFS4 (OMIM #602694), NDUFS6 (OMIM #603848), NDUFS7 (OMIM #601825), NDUFS8 (OMIM #602141), NDUFA2 (OMIM #602137), NDUFAll (OMIM #612638), NDUFAF3 (OMIM #612911), NDUFA10 (OMIM #603835), NDUFB3 (OMIM #603839), NDUFA1 (OMIM #300078) or the complex I assembly genes B17.2L (OMIM #609653), HRPAP20 (OMIM #611776), C200RF7 (OMIM #612360), NUBPL (OMIM #613621), and NDUFAF1 (OMIM #606934). A complex I deficiency results from mutation in other nuclear-encoded genes, in-cluding FOXRED1 (OMIM #613622) and ACAD9 (OMIM #611103; see OMIM #611126). A complex I deficiency with mitochondrial inheritance is associated with mutation in MTND1 (OMIM #516000), MTND2 (OMIM #516001), MTND3 (OMIM #516002), MTND4 (OMIM #516003), MTND5 (OMIM #516005), MTND6 (OMIM #516006). Features of complex I deficiency may also be caused by mutation in other mitochondrial genes, including MTTS2 (OMIM #590085).

A mitochondrial energy disorder can be selected from the group consisting of mitochondrial myopathy, encephalopathy, obesity or an obesity-related disorder.

Spermidine as well as spermidine comprising extracts can also be used in the treatment and/or amelioration of diseases associated with obesity or caused by obesity ("obesity-related disorder") like, for instance, hypertension, cardiovascular diseases, high cholesterol and type 2 diabetes, if directly associated to obesity. This is a result of the fact that spermidine is able to convert or at least supports the conversion of white adipose tissue into brown adipose tissue. Due to the reduction of adipose tissue and/or the conversion to brown adipose tissue the risk to suffer one of the aforementioned diseases associated with obesity is significantly or even substantially completely reduced.

Myopathy or encephalopathy can be caused by a genetic mutation in at least one mitochondrial complex I protein. The respective proteins which may comprise a genetic mutation are listed above.

The spermidine comprising extract can be administered orally or topically to a subject or a mammal. Therefore, the spermidine comprising extract can be formulated for oral or topical administration. These formulations may comprise next to the active ingredient spermidine other excipients which are regularly used in pharmaceutical formulations.

According to a preferred embodiment of the present invention the spermidine comprising extract is formulated in a capsule, as a tablet, as suspension, as solution, as powder, in a gel or in an ointment.

The compositions of the present invention may comprise next to the spermidine comprising extract various excipients depending on the route of administration. The use of excipients for pharmaceutically active substances is well known in the art.

Tablets of the present invention may be made by compression or molding, optionally with one or more additional ingredients. Compressed tablets may be prepared using bind-er (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of a powdered spermidine comprising composition moistened with an inert liquid diluent.

The tablets, and other solid dosage forms of the pharmaceutical compositions of the present invention, such as dragees, capsules, pills and granules, may optionally be prepared with coatings, such as enteric coatings and other coatings well known in the art. They may also be formulated so as to provide slow or controlled release of the active ingredient therein using, for example, hydroxypropylmethyl cellulose in varying proportions to provide the desired re-lease profile, other polymer matrices, liposomes or micro-spheres.

The spermidine comprising extract may be formulated to release the active ingredient only, or preferentially, in a certain portion of the gastrointestinal tract, optionally, in a delayed manner. Examples of embedding compositions which may be used include polymeric substances and waxes. The active ingredient may also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs, in addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3- butylene glycol, oils (in particular, cottonseed, groundnut, corn, germ, olive, castor and sesame oils), glycerol, tetrahydrofuryl alcohol, polyethylene glycols and fatty acid esters of sorbitol, and mixtures thereof. Besides inert diluents, the oral compositions may also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and mixtures thereof.

Dosage forms for the topical or transdermal administration of spermidine or spermidine comprising extracts include powders, sprays, ointments, pastes, creams, lotions, gels, solutions and patches. The composition may be mixed under sterile conditions with a pharmaceutically acceptable carrier and with any preservatives, buffers, or propellants which may be required. The ointments, pastes, creams and gels may contain excipients, such as animal and vegetable fats, oils, waxes, paraffins, starch, cellulose derivatives, polyethylene glycols, silicones, bentonites, silicic acid, talc and zinc oxide or mixtures thereof. Powders and sprays may contain, in addition to a composition of this invention, excipients such as lactose, talc, silicic acid, aluminum hydroxide, calcium silicates and polyamide powder, or mixtures of these substances. Sprays may additionally contain customary propellants, such as chlorofluorohydrocarbons and volatile unsubstituted hydrocarbons, such as butane and propane. Transdermal patches have the added advantage of providing controlled delivery of a composition of the present invention to the body. Such dosage forms may be made by dissolving or dispersing the composition in the proper medium. Absorption enhancers may also be used to increase the flux of the composition across the skin. The rate of such flux may be controlled by either providing a rate controlling membrane or dispersing the composition in a polymer matrix or gel.

The amount of spermidine which may be combined with excipients to produce a single dosage form will generally be that amount of the composition which produces a therapeutic effect. Generally, out of 10%, this amount will range from about 1% to about 99% of spermidine, preferably from about 5% to about 70%, most preferably from about 10% to about 30%.

According to a further preferred embodiment of the present invention spermidine is administered in an amount of 1 pg/kg body weight per day to 500 mg/kg body weight per day, preferably 5 pg/kg body weight per day to 200 mg/kg body weight per day, more preferably 10 pg/kg body weight per day to 100 mg/kg body weight per day. These amounts refer always to spermidine as active ingredient. This means that in a spermidine comprising extract the amount of spermidine comprised therein is used to calculate the amount of ex-tract or composition comprising said extract needed to obtain the required dose of spermidine to be administered.

Disclosed is a method of enhancing mitochondrial respiratory activity in a subject or a mammal comprising administering, preferably orally or topically, spermidine or a spermidine comprising extract to said subject or mammal.

Spermidine and spermidine comprising extracts as defined above can also be used non-therapeutically to enhance mitochondrial respiratory activity. It was found that spermidine is able on one hand to increase the production of ATP in mitochondria and on the other hand to reduce or to keep substantially at the same level the production of ROS. This surprising finding allows enhancing the mitochondrial respiratory activity within a cell of a subject or mammal thereby reducing the side effects expectedly caused by the increase of ATP production.

The enhancement of the mitochondrial respiratory activity may mediated via activation of mitochondrial complex I as explained and described above.

According to a further embodiment of the present invention the enhancement of mitochondrial respiratory activity increases the mitochondrial energy production (ATP) and the efficiency of mitochondrial energy production.

According to another embodiment of the present invention the enhancement of mitochondrial respiratory activity results in a reduced, substantially reduced or substantially identical formation of reactive oxygen species (ROS).

The enhancement of mitochondrial respiratory activity enhances preferably function of skeletal muscle and/or endurance and/or decreases white adipose tissue, increases formation of brown adipose tissue, transforms white adipose tissue into brown adipose tissue or enhances lipolysis.

The increase of the mitochondrial respiratory activity within a subject or mammal influences its physical status. As a result of the increased mitochondrial activity the formation and/or the strength of skeletal muscles is enhanced. The administration of spermidine has also a beneficial influence on the endurance of a subject or mammal due to a significantly increased ATP production in mitochondria.

It turned surprisingly out that spermidine is able to decrease white adipose tissue, increase formation of brown adipose tissue and to transform white adipose tissue into brown adipose tissue when administered to a subject or mammal by enhancing mitochondrial respiratory activity. This allows to mobilize adipose tissue and thus to reduce weight and the body mass index.

The mitochondrial respiratory activity is preferably enhanced in mammals like horses, camels, dogs, cats or rodents.

According to a preferred embodiment of the present invention at least one, preferably at least two, more preferably at least three, antioxidants are administered before, after or together with spermidine or the spermidine comprising extract.

It turned out that spermidine is able to increase the antioxidative effect of antioxidants when administered before, after or together with spermidine. The at least one antioxidant can be administered between 1 and 90 minutes, preferably between 1 and 75 minutes, more preferably between 1 and 60 minutes before spermidine or the spermidine comprising extract is administered.

According to another preferred embodiment of the pre-sent invention the at least one antioxidant is selected from the group consisting of ascorbic acid, α-Tocopherol, tocotrienol, retinol, polyphenols and derivatives thereof.

The polyphenol is preferably selected from the group consisting of quercetin, myricetin, catechin, theaflavin, peonidin, cyanidin, glycitein, isoflavones, resveratrol, pterostilbene, curcumin, tannins, vanillin and chlorogenic acid, where respective antioxidant is applied in daily dosage ranging from 1 mg/day to 1000 mg/day, preferably 2 mg/day to 500 mg/day, more preferably 10 mg/day to 250 mg/day.

According to a preferred embodiment of the present invention spermidine is administered in an amount of 1 pg/kg body weight per day to 500 mg/kg body weight per day, preferably 5 pg/kg body weight per day to 200 mg/kg body weight per day, more preferably 10 pg/kg body weight per day to 100 mg/kg body weight per day.

In the method of enhancing mitochondrial respiratory activity spermidine or the spermidine comprising extract is administered orally as dietary supplement or topically.

The dietary supplement of the present invention does not encompass pharmaceutical compositions and the related methods of the present invention do not encompass therapeutic treatments. The human dietary supplement described herein is a non-pharmaceutical composition and is used to enhance mitochondrial respiratory activity in a subject or a mammal. A dietary supplement, as used herein, is administered or taken by a subject or mammal more than once with the purpose of supplementing the diet to increase and/or maintain spermidine in the body at a higher level than that naturally occurring through natural or conventional meals. Additionally, dietary supplement further means an addition to the human diet in a pill, capsule, tablet, powder or liquid form, which is not part of a natural or conventional food or food product, and which effectively increases the mitochondrial respiratory activity of cells when consumed.

The increase of mitochondrial ATP production is particularly advantageous to enhance energy and muscle and/or Sports performance in a subject or a mammal. Therefore, a further aspect of the present invention relates to a (non-therapeutic) method of enhancing energy and muscle and/or sports performance in a subject or a mammal comprising administering spermidine or a spermidine comprising extract to said subject or mammal.

A further aspect of the present invention relates to a method of enhancing mitochondrial respiratory activity in a non-plant cell in vitro comprising incubating said non-plant cell in a culture medium comprising a spermidine comprising extract, wherein the spermidine comprising extract is extracted from wheat or wheat germs and wherein the culture medium comprises spermidine in a concentration ranging from 0.1 mmo1/1 to 100 mmol/l, preferably 0.1 mmo1/1 to 50 mmol/l, more preferably -0.1 mmo1/1 to 10 mmol/1.

Spermidine is able to enhance mitochondrial respiratory activity in multicellular organisms like humans and mammals as described above. However, it turned out that this beneficial effect can also be observed in individual cells cultivated, for instance, in a cell culture.

"Non-plant cells", as used herein, refers to cells which are not naturally occurring in plants and which have not been isolated therefrom. "Non-plant cells" include, for instance, human and mammalian cells as well as bacterial and fungal cells.

The cells are incubated under conditions which are usually used to cultivate the cells in vitro including the use of commonly used cell culture media supplemented with spermidine. Means and methods for the production of proteins are well known to the person skilled in the art.

The non-plant cell used in the method of the present invention is preferably a mammalian cell, preferably a human cell, a CHO cell, a HEK-293 cell, a BHK-21 cell, a NSO cell, a Sp2/0-Ag14 cell, a fungal cell or bacterial cell.

Particularly preferred fungal cells include yeast cells (e.g. Komagataella phaffii, Saccharomyces cerevisiae) or mold cells (e.g. Trichoderma). Particularly preferred

The increase of mitochondrial respiratory activity within the cultivated cells results in an increased protein production. Therefore, it is particularly preferred to enhance the mitochondrial respiratory activity to increase the yield in production of native or recombinant proteins.

With the method of the present invention polypeptides and proteins of any type can be produced. However, it is particularly preferred to produce enzymes and antibodies or functional fragments thereof, including Fc and Fab fragments as well as single chain antibodies (scFv).

### EXAMPLES:

### Example 1: Effect of spermidine on the activity of mitochondrial Complex I

Mitochondria isolated from cardyomyocytes of spermidine or sham treated 24 month old and 4 month old (young reference) C57B/6J wildtype mice were used for measurement of mitochondrial respiratory activity.

Wild-type C57BL/6 mice were purchased from Janvier Labs, France (C57BL6/J:Rj males). Supplementation of spermidine was conducted late-in-life (starting at age of 18 months). After 6 months of treatment, isolation of cardiac mitochondria and high-resolution respirometry was performed, using an isolation buffer containing 0.2% BSA and 5 mg/ml bacterial protease (Sigma-Aldrich, P8038). Hearts were quickly excised immediately after final blood collection under isoflurane anaesthesia and processed.

Optical density at 600 nm (OD600) of the final mitochondrial suspension (isolation buffer including BSA, but excluding protease) was determined by serial dilutions in a TECAN GeniusPro plate reader and served as an estimate of mitochondrial mass used for normalization. Oxygen consumption was assayed at 37 °C with an Oxygraph-2k high-resolution respirometer (Oroboros Instruments, Austria) according to the manufacturer's recommendations. OD600 equivalents of isolated myocardial mitochondria corresponding to 10-20 µg mitochondrial protein were diluted in 2 ml equilibrated measurement medium (100 mM sucrose, 20 mM K+-TES (pH=7.2), 50 mM KCl, 2 mM MgCl₂, 1 mM EDTA, 4 mM KH₂PO₄, 3 mM malate and 0.1% (v/v) BSA) within a closed and calibrated system with constant stirring.

For measurement of Complex I activity, 5 mM pyruvate and 10 mM glutamate were added as reduced substrates after initially recording residual oxygen consumption (ROX) resulting in leak respiration (LEAK), followed by sequential additions of 450 µM ADP (OXPHOS) and 10 µM cytochrome c (OXPHOS + CytC). 1.25 µM oligomycin were finally used to monitor the residual respiration (proton leak) followed by titration with FCCP (0.5 µM steps) to assess maximum respiration in uncoupled state and subsequent inhibition of respiratory activity through antimycin A. For each oxygraphic protocol, two mice were always processed in pairs (one aged control (24M) combined with either one aged spermidine-supplemented (24M+S) or one young control (5M)). The absolute oxygen concentration remained above 100 nmol/ml throughout all recordings.

Spermidine increases the activity of mitochondrial Complex I as shown in Fig. 1. In Fig. 1A representative oxygraph recordings and (B) quantification of mitochondrial respiration (only OXPHOS stage shown) from isolated cardiac mitochondria incubated with complex I supplying substrates glycine and pyruvate (Gly/Pyr) are shown. ROX, Residual respiration (State 1) in the absence of reduced substrate and ADP; LEAK, Respiration compensating for proton leak in the absence of ADP but presence of reduced substrate; OXPHOS, ADP-stimulated respiration of coupled mitochondria (quantification shown in Fig. 1B); OXPHOS+CytC, Oxphos after addition of cytochrome C. Oligomycin (Omy), FCCP, antimycin A (ama) were added as controls to block respiration by inhibition of ATP synthase, induce maximum respiration through uncoupling and block uncoupled respiration by complex III inhibition, respectively. N=8/8 (4M/24M) and N=5/5 (24M/24M+S) mice. *p<0.05, **p<0.01 (Paired Student's t-test).

The respiratory competence of cardiac mitochondria through respiratory chain complex I was increased in mice supplemented with spermidine as compared to control mice (see Fig. 1); thus, spermidine reversed an age-induced decline in mitochondrial respiratory function.

### Example 2: Effect of spermidine on the antioxidative capacity of conventional antioxidants

The synergistic effects of spermidine with some common antioxidants were measured using two step analysis of ROS reducing capacity. The first step consisted of αPROX assay as described in Fruhwirth et al. (Anal Bioanal Chem. 384(2006):703-12). Both spermidine and antioxidants were applied to reaction mix separately and in combination to assess their antioxidative capacity in reducing the fluorescence of BSA-conjugated diphenylhexatriene propionic acid (DPHPA) (represented by TEAC - trolox equivalency antioxidative capacity). This value represents mitochondria-independent reduction ROS by these substances. In the second step, the accumulation of ROS in presence of living mitochondria was measured. This allowed us to assess the ROS scavenging potential of antioxidants in combination with spermidine (and both substances individually). The difference between steps one and two in the joint effect of spermidine and conventional antioxidants against the ROS accumulation proves as a concept of the proposed synergistic mechanism.

### Example 3: Effect of spermidine on body composition

MRI analysis of spermidine or sham treated 18-24 month old and 4 month old (young reference) C57BL/6J wildtype mice was used to assess the amount of muscle, white and brown adipose tissue in each animal (Smith et al., J Magn Reson Imaging. 2013 Dec;38(6):1425-33). All MRI data were gathered using the Small Animal Imaging Core equipped with a 9.4T Bruker Magnet (BioSpec; Bruker Biospin, Billerica, Mass) with a single-channel surface coil as receiver (Bruker BioSpin). Each mouse was placed in the prone position on an animal bed individually. To regulate body temperature during measurements, animal bed was equipped with circulating warm water. Inhalation with isoflurane (1-2%) was used to keep animals anesthetized for the duration of the scans. A small-animal respiratory device was used to reduce motion artifacts. Separated water and fat signals of the underlying tissues were gathered on a voxel-wise basis across the imaging volume. This data was further used for the computation of FFs. The imaging time for each animal was kept at maximum 30 minutes. Tissues of interest were delineated based on anatomical location and the FFs were calculated across the entire voxel range. Average FFs for respective tissue type were computed for each animal.

### Example 4: Effect of spermidine on the degradation of fat (lipolysis)

Biochemical analysis of isolated blood and adipose tissue was conducted to measure the level of lipolysis by quantifying glycerol, free fatty acids and triglyceride levels using commercially available assays in sham and spermidine treated C57BL/6J wildtype mice.Free fatty acids were measured in serum using a Free Fatty Acid Quantification Kit (Abeam, Cambridge, United Kingdom) . Briefly, fatty acids were converted to their CoA derivatives. These can then be oxidized and colorimetrically analyzed. Glycerol levels were determined using a free glycerol determination kit (Sigma-Aldrich, St. Louis, MO, USA). This measurement was based on the enzymatic activity of coupled glycerol kinase and glycerol phosphate oxidase, resulting in a colorimetric product. Triglycerides were measured based on their conversion to free fatty acids and glycerol using Triglyceride Quantification Assay (Abeam, Cambridge, United Kingdom). Glycerol generated from degradation of triglycerides was oxidized leading to the generation of a product which reacts with a probe. The end product was measured using colorimetry.

### Example 5: Effect of spermidine on muscle strength and endurance

Muscle function and endurance were evaluated using rotarod performance test (Serradj et al., Behav Brain Res. 2016 Sep 1;310:126-34) on sham or spermidine treated C57BL/6J wildtype mice.

## Claims

1. Non-therapeutic use of a spermidine comprising extract in enhancing mitochondrial respiratory activity in a subject or a mammal, wherein the spermidine comprising extract is extracted from wheat or wheat germ and wherein the spermidine comprising extract comprises 10 pg/g to 500 mg/g (dry weight) spermidine.

2. Use according to claim 1, wherein the spermidine comprising extract is formulated for oral or topical administration.

3. Use according to claim 1 or 2, wherein spermidine is administered in an amount of 1 pg/kg body weight per day to 500 mg/kg body weight per day, preferably 5 pg/kg body weight per day to 200 mg/kg body weight per day, more preferably 10 pg/kg body weight per day to 100 mg/kg body weight per day.

4. Use according to any one of claims 1 to 3, wherein the enhancement of mitochondrial respiratory activity enhances function of skeletal muscle and/or endurance and/or sports performance in a subject or a mammal decreases white adipose tissue, increases formation of brown adipose tissue, transforms white adipose tissue into brown adipose tissue or enhances lipolysis.

5. Use according to any one of claims 1 to 4, wherein the spermidine comprising extract is formulated in a capsule, as a tablet, as powder, in a gel or in an ointment.

6. Use according to any one of claims 1 to 5, wherein the enhancement of the mitochondrial respiratory activity is mediated via activation of mitochondrial complex I.

7. Use according to any one of claims 1 to 6, wherein at least one antioxidant is administered before, after or together with the spermidine comprising extract.

8. Use according to claim 7, wherein the antioxidant is selected from the group consisting of ascorbic acid, α-Tocopherol, tocotrienol, retinol, polyphenols and derivatives thereof.

9. Use according to claim 8, wherein the polyphenol is selected from the group consisting of quercetin, myricetin, catechin, theaflavin, peonidin, cyanidin, glycitein, isoflavones, resveratrol, pterostilbene, curcumin, tannins, vanillin and chlorogenic acid.

10. A method of enhancing mitochondrial respiratory activity in a non-plant cell *in vitro* comprising incubating said non-plant cell in a culture medium comprising a spermidine comprising extract, wherein the spermidine comprising extract is extracted from wheat or wheat germs and wherein the culture medium comprises spermidine in a concentration ranging from 0.1 mmol/l to 100 mmol/l.

11. Method according to claim 10, wherein the non-plant cell is a mammalian cell, preferably a human cell, a CHO cell, a HEK-293 cell, a BHK-21 cell, a NS0 cell, a Sp2/0-Ag14 cell, a fungal cell or bacterial cell.

12. Method according to claim 10 or 11, wherein the mitochondrial respiratory activity is utilized for yield optimization in production of native or recombinant proteins.

## Patentansprüche

1. Nicht-therapeutische Verwendung eines Spermidin umfassenden Extrakts zur Steigerung der mitochondrialen Atmungsaktivität bei einem Subjekt oder Säugetier, wobei der Spermidin umfassende Extrakt aus Weizen oder Weizenkeimen extrahiert ist und wobei der Spermidin umfassende Extrakt 10 µg/g bis 500 mg/g (Trockengewicht) Spermidin umfasst.

2. Verwendung gemäß Anspruch 1, wobei der Spermidin umfassende Extrakt für die orale oder topische Verabreichung formuliert ist.

3. Verwendung gemäß Anspruch 1 oder 2, wobei Spermidin in einer Menge von 1 µg/kg Körpergewicht pro Tag bis 500 mg/kg Körpergewicht pro Tag, vorzugsweise 5 µg/kg Körpergewicht pro Tag bis 200 mg/kg Körpergewicht pro Tag, stärker bevorzugt 10 µg/kg Körpergewicht pro Tag bis 100 mg/kg Körpergewicht pro Tag, verabreicht wird.

4. Verwendung gemäß einem der Ansprüche 1 bis 3, wobei die Steigerung der mitochondrialen Atmungsaktivität die Funktion der Skelettmuskulatur und/oder die Ausdauer und/oder die sportliche Leistung bei einem Subjekt oder Säugetier verbessert, weißes Fettgewebe verringert, die Bildung von braunem Fettgewebe erhöht, weißes Fettgewebe in braunes Fettgewebe umwandelt oder die Lipolyse fördert.

5. Verwendung gemäß einem der Ansprüche 1 bis 4, wobei der Spermidin umfassende Extrakt in einer Kapsel, als Tablette, als Pulver, in einem Gel oder in einer Salbe formuliert ist.

6. Verwendung gemäß einem der Ansprüche 1 bis 5, wobei die Steigerung der mitochondrialen Atmungsaktivität über die Aktivierung des mitochondrialen Komplexes I vermittelt wird.

7. Verwendung gemäß einem der Ansprüche 1 bis 6, wobei mindestens ein Antioxidans vor, nach oder zusammen mit dem Spermidin umfassenden Extrakt verabreicht wird.

8. Verfahren gemäß Anspruch 7, wobei das Antioxidans ausgewählt ist aus der Gruppe, bestehend aus Ascorbinsäure, α-Tocopherol, Tocotrienol, Retinol, Polyphenolen und Derivaten davon.

9. Verfahren gemäß Anspruch 8, wobei das Polyphenol ausgewählt ist aus der Gruppe, bestehend aus Quercetin, Myricetin, Catechin, Theaflavin, Peonidin, Cyanidin, Glycitein, Isoflavonen, Resveratrol, Pterostilben, Curcumin, Tanninen, Vanillin und Chlorogensäure.

10. Verfahren zur Steigerung der mitochondrialen Atmungsaktivität in einer nichtpflanzlichen Zelle *in vitro,* umfassend das Inkubieren der nichtpflanzlichen Zelle in einem Kulturmedium, das einen Spermidin umfassenden Extrakt umfasst, wobei der Spermidin umfassende Extrakt aus Weizen oder Weizenkeimen extrahiert ist und wobei das Kulturmedium Spermidin in einer Konzentration im Bereich von 0,1 mmol/l bis 100 mmol/l umfasst.

11. Verfahren gemäß Anspruch 10, wobei die nichtpflanzliche Zelle eine Säugetierzelle, vorzugsweise eine menschliche Zelle, eine CHO-Zelle, eine HEK-293-Zelle, eine BHK-21-Zelle, eine NSO-Zelle, eine Sp2/0-Agl4-Zelle, eine Pilzzelle oder eine Bakterienzelle ist.

12. Verfahren gemäß Anspruch 10 oder 11, wobei die mitochondriale Atmungsaktivität zur Ertragsoptimierung bei der Produktion nativer oder rekombinanter Proteine genutzt wird.

## Revendications

1. Utilisation non thérapeutique d'un extrait comprenant de la spermidine dans l'amélioration de l'activité respiratoire mitochondriale chez un patient ou un mammifère, dans laquelle l'extrait comprenant de la spermidine est extrait de blé ou de germe de blé et dans laquelle l'extrait comprenant de la spermidine comprend de 10 µg/g à 500 µg/g (en poids sec) de permidine.

2. Utilisation selon la revendication 1, dans laquelle l'extrait comprenant de la spermidine est formulé pour l'administration orale ou locale.

3. Utilisation selon la revendication 1 ou 2, dans laquelle la spermidine est administrée dans une quantité de 1 µg/kg de poids corporel par jour à 500 mg/kg de poids corporel par jour, de préférence de 5 µg/kg de poids corporel par jour à 200 mg/kg de poids corporel par jour et plus préférablement de 10 µg/kg de poids corporel par jour à 100 mg/kg de poids corporel par jour.

4. Utilisation selon l'une quelconque des revendications 1 à 3, dans laquelle l'amélioration de l'activité respiratoire mitochondriale améliore le fonctionnement des muscles squelettiques et/ou l'endurance et/ou la performance sportive chez un patient ou un mammifère, réduit le tissu adipeux blanc, augmente la formation de tissu adipeux brun, transforme le tissu adipeux blanc et tissu adipeux brun ou améliore la lipolyse.

5. Utilisation selon l'une quelconque des revendications 1 à 4, dans laquelle l'extrait comprenant de la spermidine est formulé dans une capsule, sous la forme de comprimé, sous la forme de poudre, dans un gel ou dans une pommade.

6. Utilisation selon l'une quelconque des revendications 1 à 5, dans laquelle l'amélioration de l'activité respiratoire mitochondriale est assurée par l'activation du complexe mitochondrial I.

7. Utilisation selon l'une quelconque des revendications 1 à 6, dans laquelle au moins un antioxydant est administré avant, après ou avec l'extrait comprenant de la spermidine.

8. Utilisation selon la revendication 7, dans laquelle l'antioxydant est choisi au sein du groupe constitué par l'acide ascorbique, l'a-tocophérol, le tocotriénol, le rétinol, les polyphénols et les dérivés de ceux-ci.

9. Utilisation selon la revendication 8, dans laquelle le polyphénol est choisi au sein du groupe constitué par la quercétine, la myricétine, la catéchine, la théaflavine, la péonidine, la cyanidine, la glycitéine, les isoflavones, le resvératrol, le ptérostilbène, la curcumine, les tannins, la vanilline et l'acide chlorogénique.

10. Procédé d'amélioration de l'activité respiratoire mitochondriale dans une cellule non végétale *in vitro* comprenant l'incubation de ladite cellule non végétale dans un milieu de culture contenant un extrait comprenant de la spermidine, selon lequel l'extrait comprenant de la spermidine est extrait de blé ou de germes de blé et selon laquelle le milieu de culture comprend de la spermidine dans une concentration allant de 0,1 mmol/l à 100 mmol/l.

11. Procédé selon la revendication 10, selon lequel la cellule non végétale est une cellule de mammifère, de préférence une cellule humaine, une cellule CHO, une cellule HEK-293, une cellule BHK-21, une cellule NS0, une cellule Sp2/0-Ag14, une cellule fongique ou une cellule bactérienne.

12. Procédé selon la revendication 10 ou 11, selon lequel l'activité respiratoire mitochondriale est utilisée pour l'optimisation du rendement dans la production de protéines natives ou recombinantes.
